**Europäisches Patentamt**

**European Patent Office**

**Office européen des brevets**

(19)

(11) Veröffentlichungsnummer: **0 328 920 B1**

(12) **EUROPÄISCHE PATENTSCHRIFT**

(45) Veröffentlichungstag der Patentschrift :
04.12.91 Patentblatt 91/49

(51) Int. Cl.⁵: **C07C 31/22, C07C 29/136**

(21) Anmeldenummer: 89101501.8

(22) Anmeldetag : 28.01.89

(54) **Verfahren zur Herstellung von 1,2,4-Butantriol.**

(30) Priorität: 06.02.88 DE 3803581

(43) Veröffentlichungstag der Anmeldung :
23.08.89 Patentblatt 89/34

(45) Bekanntmachung des Hinweises auf die
Patenterteilung :
04.12.91 Patentblatt 91/49

(84) Benannte Vertragsstaaten :
BE CH DE FR GB IT LI NL

(56) Entgegenhaltungen :
US-A- 4 172 961

(56) Entgegenhaltungen :
THE JOURNAL OF THE AMERICAN CHEMI-
CAL SOCIETY, Band 70, September-Dezember
1948, Seiten 3121-3125, Herausgeber A.B.
Lamb et al., Columbus, Ohio, US; H. ADKINS et
al.: "The hydrogenation of esters to alcohols
at 25-150 degrees"

(73) Patentinhaber : BASF Aktiengesellschaft
Carl-Bosch-Strasse 38
W-6700 Ludwigshafen (DE)

(72) Erfinder : Müller, Herbert, Dr.
Carostrasse 53
W-6710 Frankenthal (DE)
Erfinder : Mesch, Walter, Dr.
Richinesstrasse 11
W-6700 Ludwigshafen (DE)
Erfinder : Broellos, Klaus, Dr.
Floriansring 10a
W-6104 Seeheim-Jugenheim (DE)

## Beschreibung

1,2,4-Butantriol ist ein wertvolles Zwischenprodukt mit vielfältigen Anwendungsmöglichkeiten. Da aber nur sehr aufwendige und teure Herstellverfahren für das Triol bekannt geworden sind, bleiben dem Triol die vielfältigen Einsatzmöglichkeiten, die z.B. Glyzerin gefunden hat, versagt. Es wird als Synthesebaustein bei Wirkstoffsynthesen oder zur Herstellung von Nitraten für besondere Explosivstoffe eingesetzt.

Man erhält 1,2,4-Butantriol durch Hydratisierung von Butin-2-diol-1,4 mit Hilfe von Quecksilberkatalysatoren. Aus hygienischen Gründen ist diese Umsetzung für die großtechnische Gewinnung des Triols ungeeignet. Vorteilhafter ist es, wenn man Buten-2-diol-1,4-epoxid nach den Angaben der DE-AS 2643400 und der JP-OS 59/70632, zu 1,2,4-Butantriol hydriert. Das Epoxid wird durch Umsetzung von Butendiol mit Wasserstoffperoxid erhalten. Durch eine nicht sehr selektiv verlaufende Dehydratisierungsreaktion am 1,3-Butandiol entsteht 3-Buten-1-ol. Daraus erhält man durch anschließende Hydroxylierung mit Wasserstoffperoxid am Wolfram-Katalysator in wäßriger Lösung das Triol.

In der US-PS 4410744 wird vorgeschlagen, 2,3-Epoxi-1-propanol (Glyzid) einer Hydroformylierungsreaktion zu unterwerfen und das erhaltene Oxo-Produkt katalytisch mit Wasserstoff oder mit Hydriden in 1,2,4-Butantriol zu überführen. Auch diese Synthese benötigt ein verhältnismäßig teures Ausgangsprodukt sowie mehrere Reaktionsschritte. Es wird in der Patentschrift darauf hingewiesen, daß man 1,2,4-Butantriol bisher nur im Labormaßstab durch Hydrierung von Äpfelsäure oder deren Ester hergestellt hat. Diese Laboratoriumsmethode wird in JACS 70, (1948), S. 3121 bis 3125 beschrieben. Danach gelingt es, mit der 1,5-fachen Menge an einem Kupferchromit-Katalysator, bezogen auf die Estermenge Äpfelsäurediethylester mit 50 bis 70% Ausbeute zum 1,2,4-Butantriol zu hydriern. Es wird ausdrücklich darauf hingewiesen, daß diese hohe Selektivität bei ausreichend hoher Reaktionsgeschwindigkeit nur mit im Überschuß angewendeten Katalysatoren zu erzielen ist. Bei geringeren Katalysatorkonzentrationen muß eine höhere Reaktionstemperatur eingehalten werden, was eine entsprechende Selektivitätsverminderung zu Folge hat.

Da Äpfelsäure durch Hydratisierung von Maleinsäure leicht hergestellt werden kann, stellte sich die Aufgabe, ein Verfahren zur entwickeln, das es erlaubt, 1,2,4-Butantriol auf der Basis von Maleinsäure nicht nur laboratoriumsmäßig, sondern auch in technischem Maßstab herzustellen. Es mußte insbesondere ein Weg gefunden werden, auch mit geringen Katalysatormengen Maleinsäure oder deren Ester ökonomisch vertretbar zu Butantriol zu hydrieren.

Es wurde nun gefunden, daß man bei der Herstellung von 1,2,4-Butantriol durch katalytische Hydrierung von Äpfelsäureestern an einem Kupfer enthaltenden Katalysator bei höherer Temperatur und höherem Druck besonders vorteilhafte Ergebnisse erhält, wenn man die Äpfelsäureester bei Temperaturen von 130 bis 190°C und Wasserstoffpartialdrücken von 100 bis 300 bar an dem fest angeordneten Katalysator unter weitgehender Vermeidung der Ausbildung einer Gasphase vorbeiführt.

Die für die Hydrierung geeigneten Äpfelsäureester leiten sich von Alkoholen mit 1 bis 8 Kohlenstoffatomen ab. Es können aliphatische oder cycloaliphatische Alkohole für die Esterbildung verwendet werden, wie Methanol, Ethanol, Propanol, Butanol, 2-Ethylhexanol und Cyclohexanol. Bevorzugt sind Alkohole mit verzweigter Kohlenstoffkette. Als am besten geeignete Ausgangsstoffe haben sich der Diisopropylester und insbesondere der Diisobutylester erwiesen.

Die Äpfelsäureester können in reiner Form oder als verdünnte Lösungen für die Hydrierung verwendet werden. Als Lösungsmittel geeignet sind z.B. offenkettige oder cyclische Ether, wie Diethylether oder Tetrahydrofuran, aliphatische und aromatische Kohlenwasserstoffe, die z.B. 5 bis 10 C-Atome enthalten können, andere unter den Hydrierbedingungen inerte Lösungsmittel sowie insbesondere Alkohole, wobei man zweckmäßig solche Alkohole als Lösungsmittel einsetzt, die zusammen mit der Äpfelsäure für die Veresterung verwendet wurden. Werden für die Hydrierung Lösungen der Äpfelsäureester eingesetzt, so beträgt der Lösungsmittelanteil z.B. bis zu 90 Gew.%. Zweckmäßigerweise werden Lösungen mit einem Estergehalt von 20 bis 80, insbesondere 30 bis 70 Gew.%, eingesetzt.

Erfindungsgemäß wird die Hydrierung der Äpfelsäureester nach der sogenannten Sumpf-Fahrweise ausgeführt. Bei dieser Methode wird der Ausgangsstoff in flüssiger Form oder die Ausgangsstofflösung, z.B. von unten nach oben oder von oben nach unten, an dem im Hydrierreaktor fest angeordneten Katalysator (Katalysatorbett) vorbeigeführt, wobei der Festbettkatalysator im wesentlichen ohne freien Gasraum von Flüssigkeit bedeckt ist.

Man hydriert in Gegenwart von Kupfer enthaltenden Katalysatoren, die für Carbonsäureesterhydrierung gebräuchlich sind. Diese Katalysatoren enthalten meist neben Kupfer Chrom als aktives hydrierend wirkendes Metall, wie die Adkins-Katalysatoren, die Chrom und Kupfer in annähernd äquimolaren Mengen enthalten oder die in der DE-AS 1159925 beschriebenen Katalysatoren, in denen der Chromanteil weit zurückgedrängt ist. Die Katalysatoren können neben Kupfer auch noch andere hydrierend wirkende Metalle, wie Eisen, Nickel, Kobalt, Platin, Palladium, Mangan, Molybdän, Wolfram oder Vanadium enthalten, wobei der Gehalt dieser Zusätze im Katalysator zweckmäßig 2 Gew.% nicht überschreiten sollte. Das Kupfer kann auf einem geeigneten Träger, wie Aluminiumoxid, Kieselsäuregel, Bimsstein, Magnesiumsilikat aufgetragen sein oder in geeigneter Weise mit dem Träger zusammen gefällt und nachträglich in die geeignete Form gebracht werden. Ein besonders vorteilhafter und deshalb bevorzugter Katalysator, der neben Kupfer noch Aluminium enthält, wird in der DE-OS 2445303 beschrieben.

Für die erfindungsgemäße Festbettkatalyse nach der Sumpffahrweise werden die Katalysatoren nach an sich bekannten Methoden zu Festkörpern verformt. Geeignete Formen sind z.B. Kugeln, Tabletten, Ringe oder auch einfache Strangpreßlinge. Diese werden in geeigneten druckfesten Apparaturen als Festbett angeordnet, das während der Hydrierung durch eine geschlossene Flüssigkeitsfront aus Zulauf und Reaktionsprodukt durchflossen wird. Der für die Hydrierung benötigte Wasserstoff ist bei Reaktionstemperatur und Reaktionsdruck in der Flüssigkeit gelöst oder in Form von Mikroblasen suspendiert. Es ist nicht nötig, während der Hydrierung eine aufwendige Wasserstoffkreisgasführung aufrecht zu erhalten. Dadurch gestaltet sich das erfindungsgemäße Verfahren besonders wirtschaftlich, da eine Kreisgaspumpe, die bei der an sich bekannten Esterhydrierung verwendet wird, nicht erforderlich ist.

Man hydriert bei Temperaturen von 130 bis 190°C, vorzugsweise bei 140 bis 160°C unter einem Wasserstoffdruck von 100 bis 350, vorzugsweise 200 bis 300 bar.

Die für die Hydrierung eingesetzten Flüssigkeiten sollten im wesentlichen säurefrei sein. Enthalten sie dennoch Säuren, so empfiehlt es sich, diese durch Zugabe einer anorganischen Base, wie Natriumhydroxid, Natriumbicarbonat, Natriumcarbonat, Calciumhydroxid oder auch Kaliummethylat zu neutralisieren.

Durch das erfindungsgemäße Verfahren wird bei verhältnismäßig niedriger Reaktionstemperatur eine verhältnismäßig hohe Reaktionsgeschwindigkeit erzielt. Ein weiterer Vorteil ist der, daß man einen hohen Umsatz bei einem vergleichsweise niedrigen Anfall von Nebenprodukten erreicht. Daß man nach dem Verfahren der Erfindung so vorteilhafte Ergebnisse erhält, ist überraschend, weil es anerkannte Lehrmeinung war, für Esterhydrierungen am zweckmäßigsten die Technik der Rieselfahrweise anzuwenden, wie sie z.B. in der DE-AS 1159925 (s. Spalte 1, Zeilen 1 bis 6 und Spalte 2, Zeilen 40 bis 49) beschrieben ist. Hydriert man die Äpfelsäureester jedoch nach der Rieselfahrweise, so läßt sich die Hydrierung zum 1,2,4-Butantriol nur mit einem so erheblich niedrigeren Durchsatz erreichen, daß diese Arbeitsweise als technisch brauchbare Alternative ausscheidet.

Die in dem Beispiel genannten Teile sind Gewichtsteile, sie verhalten sich zu Volumenteilen wie Kilogramm zu Litern.

Beispiel

1000 Volumenteile des nach Beispiel 1 der DE-OS 2445303 erhältlichen Kupfer und Aluminium enthaltenden Katalysators werden in Form von zylinderförmigen Tabletten von 3 mm Höhe und 3 mm Durchmesser in einen Schachtofen eingefüllt. Die Länge des Schachtofens verhält sich zu seinem Durchmesser wie 28 : 1. Vor Beginn der Hydrierung wird während des Aufheizens Isobutanol durch den Schachtofen geleitet und Wasserstoff bis zu einem Druck von 10 bar aufgepreßt, um zunächst den Katalysator vorsichtig nur in Gegenwart von reinem Isobutanol zu reduzieren. Dann wird der Wasserstoffdruck langsam von 10 bis 250 bar erhöht. Ab 150°C setzt die Reduktion des Katalysators ein, die sich durch eine merklich exotherme Reaktion anzeigt. Sobald diese abgeklungen ist und das Reduktionswasser im wesentlichen ausgetragen ist, wird die Temperatur auf 160°C erhöht und ein Wasserstoffdruck von 250 bar eingestellt. Der Isobutanol-Zulauf wird nun durch eine 30%ige Lösung von Äpfelsäurediisobutylester in Isobutanol ersetzt. Bei 160°C wird der Ofen stündlich mit 900 Volumenteilen der Esterlösung beschickt. Gleichzeitig befinden sich 2000 Volumenteile des Reaktoraustrages unter ständiger Rückführung im Umlauf. Dadurch wird die Reaktionsenthalpie abgeführt und die Reaktortemperatur im wesentlichen isotherm geführt. Während der Hydrierung werden dem Ofen stündlich 50 Normalvolumenteile Wasserstoff entnommen. Man erhält stündlich 900 Teile Reaktionsaustrag folgender Zusammensetzung :
86 Gew.% Isobutanol
2 Gew.% Butandiol-1,4
0,2 Gew.% Bernsteinsäurediisobutylester
0,5 Gew.% Butandiol-1,2
1 Gew.% Äpfelsäurediisobutylester
0,2 Gew.% Äpfelsäuremonoisobutylester
10 Gew.% Butantriol-1,2,4

Durch fraktionierte Destillation erhält man aus dem Gemisch im Vakuum farbloses 1,2,4-Butantriol von 99,8%iger Reinheit.

Man erhält das Triol mit einer Ausbeute von 60% d.Th., aber mit etwa verdoppeltem Durchsatz, wenn man für die Hydrierung eine 60 gew.%ige Lösung von Äpfelsäurediisobutylester in Isobutanol verwendet und die Hydrierung bei 170°C durchführt.

## Patentansprüche

1. Verfahren zur Herstellung von 1,2,4-Butantriol durch katalytische Hydrierung von Äpfelsäureestern an einem Kupfer enthaltenden Katalysator bei höherer Temperatur und höherem Druck, dadurch gekennzeichnet, daß man die Äpfelsäureester bei Temperaturen von 130 bis 190°C und Wasserstoffpartialdrücken von 100 bis 300 bar an dem fest angeordneten Katalysator unter weitgehender Vermeidung der Ausbildung einer Gasphase vorbeiführt.

2. Verfahren nach Anspruch 1, dadurch gekennzeichnet, daß man als Äpfelsäureester den Diisopropylester oder den Diisobutylester verwendet.

## Claims

1. A process for preparing 1,2,4-butanetriol by catalytic hydrogenation of a malic ester over a coppercontaining catalyst at elevated temperature under superatmospheric pressure, which comprises passing the malic ester at from 130 to 190°C under a hydrogen partial pressure of from 100 to 300 bar over the fixed-bed catalyst while substantially avoiding the formation of a gas phase.

2. A process as claimed in claim 1, wherein the malic ester used is the diisopropyl ester or the diisobutyl ester.

## Revendications

1. Procédé de préparation de 1,2,4-butanetriol par hydrogénation catalytique d'esters d'acide malique en présence d'un catalyseur contenant du cuivre, à température élevée et sous pression élevée, caractérisé en ce qu'on fait passer l'ester d'acide malique, à des températures de 130 à 190°C et sous des pressions partielles d'hydrogène de 100 à 300 bar, sur le catalyseur disposé en lit fixe, en évitant dans une large mesure la formation d'une phase gazeuse.

2. Procédé selon la revendication 1, caractérisé en ce qu'on utilise, comme ester d'acide malique, l'ester diisopropylique ou l'ester diisobutylique.